# EUROPÄISCHE PATENTSCHRIFT

(11) **EP 2 274 020 B1**
(45) Veröffentlichungstag und Bekanntmachung des Hinweises auf die Patenterteilung: **08.11.2017**
(21) Anmeldenummer: 08873832.3
(22) Anmeldetag: 19.12.2008
(51) Int. Cl.: A61L 15/34, A61L 15/44, A61L 15/58

(54) **HAUT- ODER WUNDAUFLAGE ZUR FEUCHTEN WUNDHEILUNG**
SKIN OR WOUND DRESSING FOR MOIST WOUND HEALING
PANSEMENT POUR LA PEAU OU POUR LES PLAIES, POUR LA CICATRISATION HUMIDE D'UNE BLESSURE

(30) Priorität: 07.04.2008 DE 102008017746
(43) Veröffentlichungstag der Anmeldung: 19.01.2011
(73) Patentinhaber: Beiersdorf AG, 20253 Hamburg (DE)
(72) Erfinder: ACHTERBERG, Volker, 22159 Hamburg (DE); HARTKOPF, Carsten, 22850 Hamburg (DE); SCHWENGLER, Helge, 21244 Buchholz (DE); FERNKVIST, Anna, 22299 Hamburg (DE)
(74) Vertreter: Hartmann, Jost
(86) Internationale Anmeldenummer: PCT/EP2008/010885
(87) Internationale Veröffentlichungsnummer: WO 2009/124578

(56) Entgegenhaltungen:
- EP-A2- 0 732 108
- WO-A1-01/97870
- WO-A1-2007/068490
- DE-A1-102004 031 955
- US-A- 2 764 976
- DATABASE WPI Week 200031 Thomson Scientific, London, GB; AN 2000-357807 XP002580829 & JP 2000 116695 A (OJI PAPER CO) 25. April 2000 (2000-04-25)

## Beschreibung

Die Erfindung umfasst eine Haut- oder Wundauflage, die aufgrund der Kombination aus wasserdampfundurchlässiger Barriereschicht und einer daran anliegenden Auflage auf Textilbasis, unabhängig von der Wasserdampfdurchlässigkeit eines Trägermaterials zur Fixierung auf der Haut, ein feuchtes Wundheilungsmilieu gewährt. In die Auflage auf Textilbasis ist zusätzlich eine Salbe in der Form inkorporiert, so dass das spontane Absorptionsvermögen des Textils erhalten bleibt.

Es ist bekannt, Wunden oder andere Hautverletzungen zum Schutz vor z.B. Infektionen und zur besseren Wundheilung mit absorbierenden Bandagen oder Pflaster abzudecken (Epidermal Wound Healing", H. I. Maibach, D. T. Rovee, editors, YearBook Medical Publishers, Inc., 1972). Bekannt ist auch, dass Wunden schneller heilen, wenn sie bedeckt werden und feucht bleiben, während sie von weiterer Abrasion geschützt sind. Ein Austrocknen der Wunde würde die Wundheilungsprozesse verzögern (Winter G.D., Formation of the Scab and the Rate of Epitehlization of Superficial Wounds in the Skin of Young Domestic Pig, Nature 193(1962)4812: 192-3, Alvarez O.M., Mertz P.M., Eagelstein W.H., The effect of occlusive dressings on collagen synthesis and reepithelialisation in superficial wounds, The Journal of Surgical Research. 35(1983): 142-8. Brett DW. A review of moisture-control dressings in wound care. J Wound Ostomy Continence Nurs. 2006 Nov-Dec;33(6 Suppl):3-8, Brett DW. Impact on exudate management, maintenance of a moist wound environment, and prevention of infection. J Wound Ostomy Continence Nurs. 2006 Nov-Dec;33(6 Suppl):S9-14)

Aus dem Bereich der Wundversorgung sind Wundverbände auf Basis von Klebemassen mit Hydrokolloiden bekannt, die gegenüber herkömmlichen Pflastern einige Vorteile aufweisen. Sie generieren ein feuchtes Wundheilungsmilieu, das die Wunde nicht austrocknen lässt und ein optimales Milieu zur schnellen Wundheilung erzeugt. Weitere Vorteile sind die Unauffälligkeit bei der Anwendung, sicheres Haftvermögen, Absorptionsvermögen von Exudat und eine gute Polsterwirkung.
So sind konturierte Wundauflagen mit einer Klebmasseschicht bestehend aus quellbaren Hydrokolloiden und wasserunlöslichen, viskosen Bestandteilen, beispielsweise Polyisobutylen, Kautschuk, Silicon oder Polyurethanelastomeren, Gegenstand der WO 92/05755.

Es existiert eine Vielzahl von Produkten zur feuchten Wundheilung, die z.B. basieren auf sog. Hydrokolloiden (s.o.), Hydrogelen, Polyurethan, Alginat oder Chitosan. Allen gemein ist der hohe bis sehr hohe Preis.

Kostengünstige textile Wundauflagen, basierend z.B. auf Baumwolle, Viskose, PE oder PP, können ein feuchtes Wundheilungsmilieu in Kombination mit oklusiven Trägermaterialien erzeugen. Dabei besteht die Gefahr der Mazeration der Haut, wenn zu viel Feuchtigkeit unter dem oklusiven Trägermaterial akkumuliert wird. Mit Mazeration ist ein flüssigkeitsbedingtes Aufweichen der wundumgebenden Haut gemeint, was zu einer aufgeweichten Hautbarriere oder Klebverlust führen kann. Eine aufgeweichte Hautbarriere kann zu Wundheilungskomplikationen, wie z.B. Infektionen, führen.

Ein Ziel der Erfindung ist, eine preiswerte textile Wundauflage bereit zu stellen, die ein feuchtes Wundheilungsmilieu erzeugt und dabei auf Trägermaterialien beliebiger, insbesondere hoher, Wasserdampfdurchlässigkeit eingesetzt werden kann.

Um die Wunde vor einer Infektion zu schützen, bringen einige Individuen antiseptische oder antibiotische Mittel auf die Wunde auf, bevor diese verbunden wird. Andere tragen z.B. die Wundheilung fördernde Mittel auf, z.B. Zinksalbe oder Dexpanthenol. Diese medizinischen Mittel können in Form einer Flüssigkeit, Fettsalbe oder einer Wasser-in-Öl Emulsion, wie z.B. einer Einreibung oder Creme, aufgetragen werden. Oft bedeutet das einen zusätzlichen Behandlungsschritt bei der Versorgung der Verletzung. Zudem neigen diese Formulierungen dazu, insbesondere bei niedriger Viskosität, zu zerlaufen oder unterhalb der Bandage auszutreten. Daher ist es relativ schwierig, die Positionierung des medizinischen Mittels in dichter Nähe zu der Wunde beizubehalten, um der Wunde die medizinische Aktivität zuzuführen. Vor allem wird dadurch aber das Haftvermögen des zur Fixierung verwendeten Trägermaterials auf der umgebenden Haut reduziert.

EP 732108 beschreibt einen strukturierten Verband mit einem oder mehreren Kohlenwasserstoffen, ausgewählt aus Petrolatum, Mineralöl, Wachsen und Fettsäuren, die kombiniert werden mit einem vernetztem Polymer in einer Menge von 0,5 bis 11,5 Gewichtsprozent und einem Verlaufmittel in der Menge von 0,5 bis 40 Gewichtsprozent um dem Problem des Wegfließen zu begegnen.
Da bei diesem Verband die gesamte Oberfläche mit den vernetzten Kohlenwasserstoffen belegt ist, verfügt er über kein spontanes Absorptionsvermögen und es kommt bei exsudierenden Wunden zum seitlichen Austritt von Exsudat. Auch das Aufsaugen von Blut oder Wundsekret ist bei dieser Wundauflage nicht möglich.

WO 03/041786 beschreibt ein Pflaster mit verschiedenen Schichten, wobei auf der wundzugewandten Seite eine strukturierte Schicht mit Senken und Spitzen vorhanden ist. Auf diese Lage kann eine Salbe (ointment) aufgetragen werden, so dass sich die Salbe in den Senken der strukturierten Schicht befindet und beim Applizieren und Tragen des Pflasters von dort an die Haut abgegeben werden kann. Dieser Produktaufbau eines Pflasters, zur Applikation einer Salbe auf die Wunde, bewirkt keine Exsudatsaugfähigkeit und ist dadurch für exsudierende oder blutende Wunden nicht geeignet.
DE 3723596 beschreibt ein Pflaster mit einer Schicht zur Auflage auf eine zu behandelnde Körperfläche und vorzugsweise mit einem Träger für die Auflageschicht und ggf. zum Befestigen an dem Körper, wobei die Auflageschicht mit Mulden mit bekannten Volumina ausgebildet ist um eine Salbe, Creme oder Puder auf die Haut zu applizieren.
Die auf die Haut aufzubringende Salbe wird dabei kurz vor Anwendung erst auf die Auflagenschicht aufgetragen.
Das Aufbringen der Salbe und das Applizieren des Pflasters sind hier zwei separate Behandlungsschritte.
US 2764976 beschreibt u.a. die Möglichkeit der Imprägnierung von Pflaster mit Petrolatum um die Klebung auf der Haut, insbesondere das Verkleben mit der Wunde, zu verringern. Als Nachteil wird dabei die hohe Feuchtigkeitsbildung und die damit verbundene Mazeration angesehen.

WO2007068490 beschreibt Wundauflagen enthaltend ein Trägermaterial mit einer darauf aufgebrachten oder darin vollständig imprägnierten Zusammensetzung, sowie eine Abdeckschicht bevorzugt mit hoher Wasserdampfdurchlässigkeit. Ein weiteres Ziel der Erfindung ist es, eine mit Creme oder Salbe dotierte textile Wundauflage bereit zu stellen, die eine einfache Einschrittanwendung von Creme bzw. Salbe und Pflaster ermöglicht und ein spontanes Absorptionsvermögen für Exsudat aufweist.
Es fehlt daher an Hautauflagen, Wundverbänden und Pflastermaterialien, die
- eine feuchte Wundheilung ermöglichen
- mit einer Salbe dotiert sind
- zu einem günstigen Preis angeboten werden können
- eine spontane Absorptionsfähigkeit aufweisen
- und keine Mazeration im Klebungsbereich zeigen.
Die erfindungsgemäße Wund- oder Hautauflage weist alle diese wünschenswerten Eigenschaften auf.

Ferner fehlt es an Wundverbänden und Pflastermaterialien, die zusätzlich den zuvor genannten Kriterien
- desinfizierende/antimikrobielle und/oder
- schmerzstillende und/oder
- entzündungshemmende Eigenschaften aufweisen.

Die erfindungsgemäße Wund- oder Hautauflage umfasst
- eine wasserdampfundurchlässige Barriereschicht und
- eine an die Barriereschicht anliegende Auflage auf Textilbasis (Textilauflage), in die eine Creme oder Salbe inkorporiert ist.

Die erfindungsgemäße Wund- oder Hautauflage, insbesondere ein Pflaster, umfasst in der bevorzugten Ausführungsform
- ein Trägermaterial, gegebenenfalls wenn gewünscht inkl. Klebmasse, beliebiger, insbesondere hoher, Wasserdampfdurchlässigkeit,
- eine wasserdampfundurchlässige Barriereschicht und
- eine an die Barriereschicht anliegende Wundauflage auf Textilbasis, in die eine Creme oder Salbe inkorporiert ist.

Die erfindungsgemäße Auflage ist bevorzugt ein Wundpflaster, das eine textile Wundauflage auf einer wasserdampfundurchlässigen Barriereschicht umfasst. Bevorzugt ist die Barriereschicht auf einem Träger angebracht. Die textile Wundauflage umfasst wiederum eine Salbe, die in der textilen Auflage inkorporiert ist und nicht aussen auf der textilen Auflage sich befindet.

Die erfindunsgemäße Gestaltung erlaubt so erst die vorteilhaften Anwendungen und bietet die dargestellten Vorteile einer feuchten Wundheilung und dennoch der Aufnahme von Exsudat.

Als Salbe (lat. Unguentum) oder Creme wird erfindungsgemäß eine halbfeste und vorteilhaft homogen aussehende Zubereitung verstanden, die zur Anwendung auf der Haut (z.B. als Wundsalbe) oder auf den Schleimhäuten bestimmt ist. Salben dienen der lokalen Wirkstoffapplikation oder der Pflege und dem Schutz der Haut, Wunden oder Schleimhäute.

Sie besteht vornehmlich aus einer fettigen Grundlage aus natürlichen oder synthetischen Stoffen und kann ein einphasiges (z. B. Paraffinum liquidum) oder mehrphasiges (Wasser-in-Öl-Emulsion, Öl-in-Wasser oder W/O/W- oder O/WO- Emulsionen) System sein. Wirkstoffe oder Arzneistoffe können in Lösung oder Dispersion in der Salbe oder Creme eingearbeitet sein. Die Freisetzung von Wirkstoffen aus der Salbe ist erfindungsgemäß möglich und bevorzugt.

Im erfindungsgemäßen Sinne zählen zum Begriff Salbe
- Salben in engerem Sinn, einphasig,
- Cremes,
- Gele, Lotionen und
- Pasten

Als Salbe, die insbesondere bevorzugt wasserfrei ist, wird insbesondere Petrolatum oder Mischungen umfassend Petrolatum verwendet. Neben oder anstelle von Petrolatum kann die Salbe bevorzugt auch Mineralöl, Ceresin und/oder Lanolinalkohol umfassen.

Wirkstoffe können in der Salbe bzw. Creme enthalten sein. Wirkstoffe sind insbesondere natürliche Zusatzstoffe und/oder viskositätserhöhende Stoffe.

Vorteilhaft kann die Salbe entzündungshemmende, wundheilungsstimulierende, schmerzlindernde oder antimikrobielle Wirkstoffe enthalten. Beispielsweise kann die Salbe Dexpanthenol, oder natürliche Zusatzstoffe wie Honig, Kamille oder Aloe Vera enthalten. Weitere mögliche Wirkstoffe sind Vitamine wie z.B. Vitamin A, C oder E, Wachstumsfaktoren z.B PDGF, Zucker oder Polysacharide wie z.B. Glucose oder Hyaluronsäure, Mineralstoffe wie z.B. Zink, Aminosäure und deren Derivate wie z.B. Arginin oder Kreatin. Weiter mögliche Wirkstoffe sind Cyclooxogenas-Inhibitoren wie zum Beispiel Acetylsalicylsäure, Nichtsteoridales Antirheumatikum (NSAIDs) wie Diclofenac, Ibuprofen, Ketaprofen oder Indometacin. Weitere mögliche Wirkstoffe sind Lokalanästhetika wie zum Beispiel Benzocain, Procain, Lidocain oder Prilocain. Viskositätserhöhende Stoffe wie Fette oder Wachse können auch enthalten sein. Die Wundauflage kann auch Chitosan, Klorhexidin, Jod, Silber, Triclosan, Likokalchon, Polidocanol, Zinkoxid oder Fungiziden enthalten.

Als Salbe kann vorteilhaft eine im Handel erwerbliche Salbe, wie beispielsweise Aquaphor® (mit und ohne Dexpanthenol) oder Vaseline® verwendet werden.

Wasserfreiheit der Salbe ist aufgrund der damit verbundenen besseren Stabilität und Lagerfähigkeit als auch aufgrund einer besseren Herstellbarkeit des Pflasters von Vorteil.

Auch kosmetische Wirkstoffe zur Pflege der Haut können in der Salbe enthalten sein.

In einer bevorzugten Ausführungsform umfasst die Salbe ein oder mehrere Wirkstoffe gewählt aus Stoffen der Analgestika, Antirheumatika, Desinfizientia, Antiseptika und/oder Lokalanesthetika.
Bevorzugt wird die Auflage als Lokalanästhetikum formuliert.
Die Verwendung einer Wund- oder Hautauflage umfassend eine
wasserdampfundurchlässige Barriereschicht und eine daran anliegende Auflage auf Textilbasis, in die eine Salbe oder Creme inkorporiert ist, die einen oder mehrere Wirkstoffe gewählt aus der Gruppe der Analgestika, Antirheumatika, Desinfizientia, Antiseptika, Antibiotika, Antimykotika und/oder Lokalanesthetika zur Herstellung eines Analgestikum, Antirheumatikum, Desinfizientium, Antiseptikum, Antibiotikum, Antimykotikum und/oder Lokalanesthetikum umfasst ist bevorzugt.

Besonders die Verwendung der Auflage umfassend ein oder mehrere Wirkstoffe aus der Gruppe der Lokalanesthetika zur Herstellung eines Lokalanesthetikum ist vorteilhaft und bevorzugt.

Ebenso die Verwendung einer Auflage umfassend mindestens einen Wirkstoff gewählt aus den entzündungshemmenden oder wundheilungsfördernden Wirkstoffe zur lokalen Schmerzlinderung und Entzündungshemmung ist eine besonders bevorzugte Anwendung.

Vorteilhaft hat sich darüber hinaus die Verwendung einer Auflage umfassend mindestens einen Wirkstoff gewählt aus der Gruppe Antiseptika, Antibiotika oder Antimykotika zur lokalen Wunddesinfektion und/oder Verminderung der Keimbesiedlung herausgestellt.

Vorteilhaft kann die Auflage entzündungshemmende, wundheilungsstimulierende, schmerzlindernde oder antimikrobielle Wirkstoffe enthalten.

Die Wirkstoffe sind vorteilhaft in der Salbe enthalten, können aber auch in der Textilauflage selbst enthalten sein. Das Einbringen der Wirkstoffe in die Auflage kann daher entweder
- als Bestandteil der Salbe oder
- durch Besprühen bzw. Tränken der Textilauflage mit einer Wirkstofflösung und gegebenenfalls nachfolgendem Entfernen des Lösungsmittels (z.B. durch Trocknung) erfolgen.
Im zuletzt genannten Fall wird die Salbe als zweiter Schritt auf die trockene Textilauflage aufgebracht. Die Lösung kann einen Wirkstoff oder mehrere Wirkstoffe zu einem Anteil von 0,01 bis 10 Gew.% je Wirkstoff, bevorzugt 0,1 bis 5 Gew.% je Wirkstoff, bezogen auf die Gesamtmasse der Lösung, enthalten.

Beispielsweise kann die Auflage entzündungshemmende oder wundheilungsfördernde Wirkstoffe wie Zinkacetat 2H₂O, Zinksulfat 7H₂O, Allantoin, Aloe vera, Arnika, Bisabolol, Calendula, Chamomilae, Dexpanthenol, Enzyminhibitoren , Hamamelis, Harnstoff, Honig/Manukahonig, Hyaluronsäure, Johanniskraut, Kamillenextrakt, Polidocanol, Propolis, Vitamine oder Provitamine, Wachstumsfaktoren, z.B. PDGF, Weizenkeimextrakt, Zinkoxid, Vitamin A, Vitamin C, Vitamin E, Chitosan Capsicum annuum L. und Derivate, Benzydamin, Benzylnicotinat, Bufexamac, Diclofenac, Etofenamat, Flufenaminsäure, Heparinoide Ibuprofen, Indometacin, Ketoprofen, Naxoprophen, Piroxicam , Salicylsäure und ihre Derivate,Teniposid, 2-Hydroxybenzoesäure, Acetylsalicylsäure und Derivate, Acid. silicicum D8, Aconitum (Eisenhut), Aconitum D3, Aescin (Rosskastanie), D4 ("Hundspetersilie"), Ammoniumbituminosulfonat (ICHTHYOL®), Arnica D3, Arnica mont. D4, Arnica montana ex herba rec. ad usum ext, Ausz. aus Guajakholz, Auszug aus Arnica montana, Balsamum peruvianum (Perubalsam),Beinwellwurzel-Fluidextrakt, Benzylnicotinat, Calendula ad usum ext., Calendula D3, Calendula Ø, Calendula offic. D3, Calendula officinalis Ø, Campher, Chamomilla, Colchicum e seminibus D4,Conium D2,Cort. Heisteriae,Cort. Salicis,D-Campher,Delphinium staph (Stefanskörner), Delphinium staph. D4, Dexpanthenol, Diclofenac-Diethylamin, Diclofenac-Natrium, Diethylazan-Salz, Dimethylsulfoxid, Echinacea ang.,Echinacea Ø, Echinacea purp., Echinacea purp. Ø, Echinacea purpurea, Planta tota Ø, Echtem Goldrutenkraut, Etofenamat, Extr. Flor Calendulae spiss, Extr. Fol Digitalis fluid, Extr. Fol Hyoscyami fluid, Extr. Herba Conii maculate, Extr. Rad. Petasit. Spiss, Extr. Rhiz. Podophylli fluid., Extr. Semen Colchici fluid, Ferrum phosphoricum D10, Fichtennadelöl, Flufenaminsäure, Fol. Betulae, Guaiacum Ø (Guajakharz), Guajakholz-Trockenextrakt, Hamamelis D4 ("Zaubernuss"), Harpagophytum procumbens, Helianthus ann. D4,Hepar sulf. D8, Hepar sulfuris, Heparin-Natrium, Huminsäuren, Hydroxyethylsalicylat, Hypericum, Ibuprofen, Indometacin, Isobornylacetat, Kalium bichrom. D8, Kalium sulf. D8, Kalium-Eisen-Phosphat-Citrat-Komplex, Ketoprofen, Lachesis mutus D8, Latschenkieferöl, Levisticum, Rad. sicc. H 10%, Levomenthol, Mercurialis perennis 2b Ø, Mercurius bijodatus D5, Mercurius solub. Hahnem, Mercurius solub. Hahnem. D8, Methylnicotinat, Methylsalicylat (Salicylsäuremethylester), Millefolium, Myrtecain, Naloxon HCl, Natriumthiosulfat, natürl. Eifelfango, Nicoboxil, Paracetamol, Pl. tota rec, Quarz, Rad. Harpagophyti (Teufelskralle), Rad. Harpagophytum procumbens, Resina Laricis (Terebinthina laricina), Rhus toxicodendron D6, Ruta D6 (Weinraute), Salicylsäure, Schwefel, Stibium metallicum praeparatum, Stibium sulfuratum nigrum D1, Symphytum (Beinwell), Symphytum D6, Symphytum offic. e rad. D6, Teufelskrallenwurzel-Extrakt, Tilidin-HCl 0,5H₂O, Tilidinphosphat,Toxin der Vipera ammodytes, Tramadol-HCl, Extrakt aus Brennesselblättern, Extrakt aus Roßkastaniensamen, Extrakt aus Weidenrinden, Extrakte der Zitterpappelrinde und -blättern und/oder Triclosan enthalten.

Besonders bevorzugt sind Dexpanthenol, Allantoin, Bisabolol, Harnstoff, Honig, z.B. Manukahonig, Hyaluronsäure, Licochalcon, insbesondere Licochalcon-A, Polidocanol, Propolis, Zinkoxid, Vitamine und/oder Provitamine wie z.B. Vitamin A, C oder E in der Auflage enthalten.
Derivate sowie verwandte und ähnliche Stoffe, auch in Kombination bzw. in Kombination mit anderen Substanzen können auch enthalten sein. Aloe Vera, Calendula, Kamille, Hamamelis, Johanniskraut, Weizenkeim, Eukalyptus und deren Extrakte oder Wirkstoffkombinationen mit diesen Stoffen können auch enthalten sein. Weitere mögliche wundheilungsfördernde Wirkstoffe sind sonstige pflanzlichen wundheilungsfördernden Substanzen oder Substanzgemische bzw. Pflanzenextrakte. Der Einsatz von einem oder einer Kombination von den aufgelisteten Stoffen fördern neben der feuchten Wundheilung zusätzlich die Wundheilung. Wundheilungsfördernde Stoffe können zu einem Anteil von 0,1 bis 10 Gew.%, bevorzugt von 0,5 bis 5 Gew.%, insbesondere im Bereich von 1 bis 4 Gew.%, bezogen auf die Gesamtmasse der Salbe, in der Salbe/Creme vorhanden sein.

Flüssige Stoffe wie z.B. Honig oder Reibungen können auch direkt auf die Textilauflage, während der Herstellung appliziert werden, um eine feuchte Wundheilung kombiniert mit dem wundheilungsfördernden Effekt zu bieten.

Wachstumsfaktoren, wie z.B. PDGF, können auch in der Auflage enthalten sein, um die Wundheilung zu fördern. Beruhigende Wirkstoffe wie zum Beispiel Isotretionin und Zinksulfat können zu einem Anteil von bevorzugt 0,0005 bis 0,1 Gew.%, bevorzugt von 0,001 bis 0,01 Gew.%, enthalten sein.

Weitere mögliche in der Auflage, bevorzugt als Bestandteil der Salbe, enthaltene Wirkstoffe sind Lokalanästhetika, die zur Oberflächenanästhesie geeignet sind, wie z.B. Arsenicum album D12 (weißes Arsen), Articain, Articain-HCl mit Epinephrin HCl, Atropium sulf. D5, Benzalkoniumchlorid, Benzocain, Bupivacain, Bupiviacin-HCl, Chlorethan, Cinchocain, Dibucain, Etidocain, Fomocain, Formica rufa D12 (rote Waldameise), Hypericum perf. D5, Lidocain, Lidocain-HCl, Mepivacain, Mepivacain-HCl, Methyl-4-hydroxybenzoat, Oxybuprocain, Oxybuprocain-HCl, Prilocain, Procain, Procain-HCl, Procain-HCl mit Koffein, Propyl-4-hydroxybenzoat, Quinisocain, Ropivacain, Ropivacain-HCl, Sulfur D12, Tetracain, Tetracain-HCl mit Macrogollaurylether und/oder Liquorize-Extrakte.

Ganz besonders vorteilhaft können Wirkstoffe zur lokalen Schmerzlinderung wie zum Beispiel Benzocain, Procain, Lidocain oder Prilocain in der Auflage enthalten sein.

Der Einsatz von Lokalanästhetika bietet einen zusätzlichen Nutzen einer schnellen Schmerzlinderung der verletzten Hautstelle. Lokalanästhetika können zu einem Anteil von 0,1 bis 4 Gew.% in der Salbe bzw. Textilauflage eingesetzt werden, bevorzugt zu einem Anteil von 0,5 bis 3 Gew.%, insbesondere von 1 bis 2,5 Gew.%, bezogen auf die Gesamtmasse der Salbe bzw. Auflage.

Andere besonders bevorzugte Wirkstoffe zur lokalen Schmerzlinderung sind Articain, Atropium sulf. Benzalkoniumchlorid, Bupivacain, Chlorethan, Cinchocain, Dibucain, Etidocain, Fomocain, Mepivacain, Oxybuprocain, Propyl-4-hydroxybenzoat, Quinisocain, Ropivacain, Tetracain oder Formica rufa. Diese können in der Textilauflage/Salbe alleine oder in Kombination mit anderen Stoffen enthalten sein. Damit sind auch deren Derivate sowie verwandte und ähnliche Stoffe wie z.B. Salze, wie Lidocain-HCl oder Procain-HCl, gemeint. Die oben genannten Stoffe zur lokalen Schmerzlinderung können auch mit anderen Wirkstoffen wie z.B. wundheilungsfördernden Stoffen, kombiniert werden, um zusätzliche Leistungen neben der feuchten Wundheilung des Pflasters zu bieten.

Weiter besonders vorteilhafte Wirkstoffe sind Cyclooxygenas-Inhibitoren zur Schmerzlinderung und Entzündungshemmung, wie zum Beispiel Acetylsalicylsäure oder nichtsteoridales Antirheumatikum (NSAIDs) wie zum Beispiel Diclofenac, Ibuprofen, Ketoprofen, Benzydamin, Benzylnicotinat, Bufexamac, Etofenamat, Flufenaminsäure, Heparinoide, Naxoprophen, Piroxicam, Teniposid oder Indometacin. Deren Derivate sowie verwandte und ähnliche Stoffe auch in Kombination bzw. in Kombination mit anderen Substanzen können in der Textilauflage/Salbe enthalten sein um einen zusätzlichen Schmerzlinderungseffekt zu bieten. Cyclooxygenas-Inhibitoren können zu einem Anteil von 0,1 bis 3 Gew.%, bevorzugt zu einem Anteil von 0,5 bis 2,5 % enthalten sein. Pflanzliche schmerzstillende Mittel wie zum Beispiel Aescin, Guajakholz, Rhiz. Podophylli, Herba Conii, Fol Hyoscyami, Fol Digitalis, Echinacea purpurea, Eschenrinde, Delphinium staph, Campher, Balsamum peruvianum, Beinwellwurzel, Goldrutenkraut und Aethusa sowie Extrakte und Kombination davon können auch als sehr vorteilhaft in der Auflage enthalten sein, vorteilhaft zu einem Anteil von 0,1 bis 5 Gew.%, insbesondere vorteilhaft zu einem Anteil von 0,5 bis 3 Gew.%, bezogen auf die Gesamtmasse der Textilauflage oder Salbe.

Die Auflage kann auch antiseptische, antibiotische oder antifungizide Wirkstoffe enthalten. Eine antiseptische Wirkung der bevorzugt als Wundauflage dargebotenen Auflage beugt Infektionen der Wunde vor und bietet dadurch einen zusätzlichen Nutzen der erfindungsgemäßen Auflage.

Antibiotische und antifungizide Wirkstoffe, zu denen unter anderem die Aminoglykoside gehören, beugen Infektionen vor, in dem sie das Wachstum der Mikroorganismen verhindern. Als vorteilhaft zu nennen sind Alkohole (z.B. 1-Propano, I2-Propanol), antimikrobiellen Metalle, beispielsweise Silber, oder deren Salze, Chlorhexidin, Octenidinhydrochlorid, Polyhexanid, Povidon-Jod, Taurolidin, 2,2'-Methylenbis(6-brom-4-chlorphenol), 2-Biphenylol (Instrumente), 3,5-Dibrom-4-hydroxybenzolsulfonsäure, 5-Chlor-2-hydroxybenzoesäure, Aluminiumacetat-Tartrat, Benzalkoniumchlorid, Benzylalkohol, Biphenyl-2-ol, Clorofen, Butan-1,3-diol, Chinolin-8-olsulfat, Chinolinolsulfat-Kaliumsulfat, Chlorhexidinbis(D-gluconat), Chlorhexidindigluconat, Cocospropylendiamin, Didecyldimethylammoniumchlorid, Ethacridinlactat 1H₂O, Glucoprotamin, Glutaral, Kaliumthiocyanat, Mecetroniumetilsulfat, Methyl-4-hydroxybenzoat, Octenidin-2HCl, Phenoxyethanol, Polihexanid, Povidon-Iod, Propyl-4-hydroxybenzoat, Tosylchloramid-Natrium 3H₂O, Undecylensäure, Wasserstoffperoxid, Bifonazol, Ammoniumbituminosulfonat, Bacitracin, Benzoylperoxid, Chinolin-8-olsulfat, Chloramphenicol, Chlortetracyclin-HCl, Clindamycin, Clindamycin-2-dihydrogenphosphat, Clioquinol, Erythromycin, Framycetinsulfat (neomycin B), Fusidinsäure 0,5H₂O, Gentamycinsulfat, Gereinigtes Terpentinöl, Imiquimod, Isotretinoin , Lärchen terpentin, Meclocyclin (5-sulfo-2-hydroxybenzoat), Metronidazol Miconazolnitrat, Mupirocin, Nadifloxacin, Natriumbituminosulfonat (ICHTHYOL®-Natrium), Natriumfusidat, Neomycinsulfat, Oxytetracyclin-HCl, Podophyllotoxin, Retapamulin, Sulfadiazin-Silber, Tetracyclin-HCl, Tretinoin, Trockenextrakt aus Melissenblättern, Tyrothricin. Besonders vorteilhaft kann Chitosan, Chlorhexidin, Povidon-Iod, Silber oder Sulfadizin-Silber, Triclosan, Octenidinhydrochlorid, Polyhexanid, Taurolidin oder Fungiziden enthalten.
Derivate, ähnliche Stoffe und Kombinationen davon sind damit auch erfindungsgemäß mit umfasst. Pflanzliche antiseptische Stoffe wie gereinigtes Terpentinöl oder Lärchenterpentin können ebenfalls als Wirkstoffe in der Textilauflage/Salbe enthalten sein. Die Auflage kann insbesondere Chitosan enthalten, vorteilhaft bis zu 50 %, vorteilhaft bis zu 20 %.
Sonstige antiseptische Wirkstoffe können zu einem Anteil von 0,1 bis 5 Gew.% in der Textilauflage/Salbe enthalten sein, bevorzugt zu einem Anteil von 0,5 bis 3 Gew.%, bevorzugt zu einem Anteil von 0,5 bis 2 Gew.%, bezogen auf die Gesamtmasse der Textilauflage/Salbe. Weitere antiseptische Wirkstoffe wie z.B. Ethacridinlactat können zu einem Anteil von 0,05 bis 1 Gew.% enthalten sein, vorteilhaft zu einem Anteil von 0,1 bis 0,5 Gew.%.

Besonders vorteilhafte Aminoglykoside wie z.B. Neomycinsulfat, Metronidazol, Erythromycin, Framycetinsulfat oder Gentamycinsulfat können ebenfalls in der Auflage enthalten sein. Ein Anteil von 0,1 bis 3 Gew.% von Aminogykosid in der Textilauflage/Salbe ist vorteilhaft. Gentamycinsulfat wird vorteilhaft zu einem Anteil von 1,5 bis 2,5 Gew.% eingesetzt. Framycetinsulfat wird bevorzugt zu einem Anteil von 0,1 bis 0,5 Gew.%, jewiels bezogen auf die Gesamtmasse der Textilauflage bzw. Salbe, eingesetzt.

Zucker oder Polysacharide wie z.B. Glucose kann in der Auflage/Salbe enthalten sein um die Flüssigkeitsaufnahme zu verbessern oder um Stoffe aus dem Wundbett zu ziehen.

Aminosäure und deren Derivate wie z.B. Arginin oder Kreatin können ebenfalls als Wirkstoffe umfasst sein.

Wirkstoffe zur schnelleren Abheilung von Herpes-Bläschen können in der Auflage/Salbe enthalten sein. Beispiele sind Heparin-Natrium, Foscarnet-Natrium, Tromantadin, Idoxuridin, Dimethylsulfoxid, Pencicolvir und Aciclovir. Einen virustatischen Wirkstoff bietet neben der feuchten Wundheilung eine zusätzliche Wirkung für eine schnellere Abheilung von Herpes-bläschen-ausbrüchen. Virustatika können vorteilhaft zu einem Anteil von 1 bis 10 Gew.% enthalten sein, bevorzugt zu einem Anteil von 3 bis 8 Gew.%. Pflanzliche Virustatika wie z.B. Extrakte aus Melisseblättern können zu einem Anteil von 1 bis 20 Gew.%, vorteilhaft zu einem Anteil von 5 bis 15 Gew.% enthalten sein.

Die Wirkstoffe sind vorteilhaft zu einem Anteil von 0,001 - 10 Gew.%, bevorzugt 0,01 - 5 Gew.%, insbesondere bevorzugt 0,05-1 Gew.%, bevorzugt 0,01-0,5 Gew.%, bezogen auf die Gesamtmasse der Salbe oder Textilauflage, in der Auflage enthalten.

Bevorzugt wird die Wund- oder Hautauflage umfassend eine wasserdampfundurchlässige Barriereschicht und eine daran anliegende Auflage auf Textilbasis, in die eine Salbe oder Creme inkorporiert ist, die einen oder mehrere Wirkstoffe gewählt aus der Gruppe der Analgestika, Antirheumatika, Desinfizientia, Antiseptika, Antibiotika, Antimykotika und/oder Lokalanesthetika zur Herstellung eines Analgestikum, Antirheumatikum, Desinfizientium, Antiseptikum, Antibiotikum, Antimykotikum und/oder Lokalanesthetikum angewendet.

Die Salbe bzw. Creme ist in der textilen Wund- oder Hautauflage inkorporiert. Textil bedeutet erfindungsgemäß Gewebe, Gewirke oder Vliese und auch perforierte oder atmungsaktive Kunststofffolien sind erfindungsgemäß als Textil einsetzbar.

Die Auflage auf Textilbasis, der Textilauflage, besteht vorteilhaft aus sogenannten non woven (Vliesen), insbesondere aus Viskose, Polyester und/oder Polypropylen (PP), wobei PP besonders bevorzugt ist.
Textilbasis für die Auflage kann auch Filz oder Gewebe sein.
Die Dicke der Auflage liegt vorzugsweise im Bereich von etwa 0,1 bis 2 mm, besser 0,5 bis 1,5 mm (DIN EN ISO 9073-2), mit einem bevorzugten Flächengewicht von etwa 50 bis 200 g/m², bevorzugt 100 bis 140 g/m². Sie kann einlagig sein oder aus mehreren Lagen gleicher oder unterschiedlicher Zusammensetzung aufgebaut sein.
Der Bereich der Textilauflage wird erfindungsgemäß über die Dicke definiert. Es ist dabei eine bestimmte Dicke der Wundauflage zu wählen, um den Effekt des feuchten Milieus und gleichzeitiger Wundsekretabsorption zu gewährleisten.

Die Größe der Textilauflage kann je nach zu behandelnder Haut bzw. Wunde variieren. Für kleinere Wunden liegt die Größe der erfindungsgemäßen Wundauflage bevorzugt im Bereich von 18 x 30 mm², insbesondere im Bereich 12 x 25 mm². Für größere Wunden können Größen der Wundauflage im Bereich von 40 x 50 mm² gewählt werden, insbesondere 30 x 40 mm². Die Form der Wundauflage kann je nach Wundform gewählt werden, z.B. rund, oval, oder rechteckig

In Bezug auf die bevorzugten Größen der Textilauflage ist die darin inkorporierte Menge an Salbe bevorzugt im Bereich von 0,01 bis 0,2 g, insbesondere im Bereich von 0,04 - 0,15 g, bevorzugt im Bereich von 0,06 - 0,1 g, zu wählen.

Als Barriereschicht werden Folien gewählt, die wasserdampfundurchlässig sind. Vorteilhaft sind die Folien gegenüber der Salbe und den darin enthaltenen Komponenten, wie insbesondere Petrolatum, inert. D.h. die Folien nehmen das Fett, die Inhaltsstoffe der Salbe nicht auf. Besonders geeignete Materialien als Barriereschicht sind z.B. Polyethylentherephthalat (PET) oder Polyamid.
Die Barriereschicht ist vorteilhaft mit dem Vlies laminierbar. Es kann eine thermische Laminierung durch Schweißung (die Folie wird auf das Vlies der Auflage aufgeschmolzen) oder eine Verklebung, z.B. mittels Polyurethankleber (härtet aus) stattfinden.

Als wasserdampfundurchlässig wird erfindungsgemäß eine Folie mit einer "water vapor transmission rate (WVTR)" von weniger als 300 g/m² in 24h, insbesondere weniger als 100 g/m² 24h und besonders bevorzugt von weniger als 50 g/m² 24h angesehen.

Die Dicke der Barriereschicht liegt bevorzugt zwischen 5 und 30 µm, insbesondere im Bereich von 10 bis 15 µm, vorteilhaft 12 µm. D.h. sie ist dünn genug um flexibel zu sein um keinen Diskomfort beim Anwender zu verursachen und dick genug um als Wasserdampfbarriere zu wirken.
Bevorzugt ist die Barriereschicht aus ästhetischen Gründen transparent oder weiß gestaltet. Die Barriereschicht kann, wenn sie ohne Träger angewendet wird, auch beliebig bedruckt oder farbig sein um den individuellen ästhetischen Vorstellungen der Anwender zu genügen.

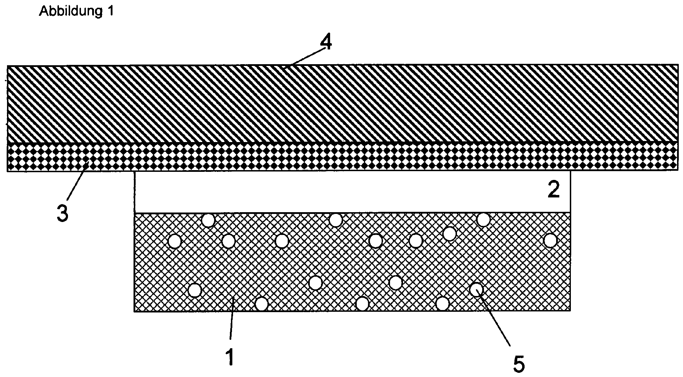

Durch die textile Auflage wird die Salbe damit in der Auflage inkorporiert. D.h. die Salbe befindet sich in den Leeräumen des textilen Materials. Inkorporieren bedeutet erfindungsgemäß beinhalten, einverleiben. D.h. die Salbe ist in der Wundauflage und nicht oberflächlich aufgetragen, wie es in der Abbildung 1 auch dargestellt ist.

Die Auflage bestehend aus wasserdampfundurchlässiger Barriereschicht, darauf aufgebrachter textiler Auflage und darin enthaltener Salbe gewährleistet eine einfache und effektive Hautpflege oder Wundbehandlung. Die Auflage kann als Pad oder Abdeckung auf die Haut oder Wunde aufgebracht werden.
Eine zusätzliche Fixierung der Auflage auf der Haut ist individuell möglich, beispielsweise mit Heftstreifen oder Pflaster, wie Leukostrip.

Bevorzugt umfasst die Auflage ein Trägermaterial. Das Trägermaterial kann ein für Pflaster bekanntes Material sein. Als Trägermaterialien eignen sich alle starren und elastischen Flächengebilde aus synthetischen und natürlichen Rohstoffen. Bevorzugt sind Trägermaterialien, die nach Applikation der optionalen Klebemasse so eingesetzt werden können, dass sie Eigenschaften eines funktionsgerechten Verbandes erfüllen. Beispielhaft sind Textilien wie Filz, Gewebe, Gewirke, Gelege, Vliese, Laminate, Netze, non woven, Folien, vorteilhaft perforierte Folien, Schäume und Papiere aufgeführt. Vorteilhafte Materialien sind zum Beispiel Polypropylene, Polyethylene, Polyetherester, Polyamid, Baumwolle, Viskose oder Mischungen davon. Wenn das Trägermaterial eine Folie ist, hat es eine Dicke von bevorzugt 10 bis 100 µm. Weiter können diese Materialien vor- bzw. nachbehandelt werden. Gängige Vorbehandlungen sind Corona und Hydrophobieren; geläufige Nachbehandlungen sind Kalandern, Tempern, Kaschieren, Stanzen und Eindecken. Das Trägermaterial kann auch aus einem Laminat bestehen, wie zum Beispiel ein Laminat von Polypropylene und Polyethylene.
Die Trägermaterialien können nach Bedarf luft- und wasserdampfdurchlässig und/oder wasserundurchlässig gestaltet sein.

Optional ist die Trägerschicht mit einer Klebemasse ausgerüstet. Die Klebematrix kann auf der Trägerschicht oder -folie aufgebracht sein, wie es aus dem Stand der Technik bekannt ist. Dabei wird die Klebmatrix einseitig mit dem Trägermaterial abgedeckt und als Verbundfolie appliziert. Je nach verwendetem Trägermaterial können dadurch die Wasserdampfdurchlässigkeit, die Festigkeit der Wundabdeckung, die Polsterung gegen Druck und andere physikalische Eigenschaften der Wundabdeckung gesteuert werden.

Die Trägerschicht mit Klebmatrix ist vorteilhaft größer als die Barriereschicht und die Wundauflage konzipiert und überlappt diese, so dass eine Applikation der textilen Wundauflage auf einer Wunde mit gleichzeitiger Klebung des Pflasters an der umgebenden Haut erfolgt (siehe Abbildung 1)

Die Klebmasse kann aus unterschiedlichen Massen, wie beispielsweise Acrylaten, Kautschuk (natürlich oder synthetisch), Polyurethan, Silikon oder Hydrokolloiden gewählt werden.

Die Auflage kann in einer Ausführungsform einen Ring oder ähnliche dicke Polsterung als Druckschutz um die Wundauflage herum enthalten oder mit einer solchen Polsterung kombiniert werden.

Die Kombination aus Träger, wasserdampfundurchlässiger Barriereschicht und textiler Wundauflage alleine, stellt für sich bereits eine gute Möglichkeit der feuchten Wundheilungsmöglichkeit dar. Die Kombination mit einer Salbe fördert das feuchte Wundheilungsmilieu und dient der Haut- und Wundpflege.
Wesentlich ist hierbei, dass sich die Salbe innerhalb der textilen Wundauflage befindet und die Auflage eine weiterhin bestehende Absorption von Exsudat aufweist

Ist das Trägermaterial selbst nicht klebend ausgeführt, ist eine Befestigung der Auflage über zusätzliche Klebestreifen möglich.

Die Anordnung der erfindungsgemäßen Auflage ist bevorzugt eine zur Haut bzw. Wunde gerichtet Textilauflage, in die eine Salbe inkorporiert ist, eine sich daran anschließende, eine daran anliegende Barriereschicht, die wiederum auf dem Trägermaterial bevorzugt über Klebemasse befestigt ist.

Die Abbildung 1 zeigt bevorzugte Anordnungen der Träger-, Barriere- und Wundauflageschichten in skizzenhafter Darstellung.

Auf einen wasserdampfdurchlässigen Träger (4) ist eine Klebmasse (3) aufgebracht, auf die ein kleineres Laminat aus Barriereschicht (2) und textiler Wundauflage aufgebracht ist. In letzterer ist eine Salbe (5) inkorporiert.
Optional kann zusätzlich zur Salbe ein Wirkstoff in die textile Wundauflage inkorporiert sein.

Die Barriereschicht erfüllt dabei insbesondere zwei Zwecke. Sie bildet eine Barriere zwischen Salbe und der bevorzugt vorhandenen Klebemasse des Trägers und gewährleistet damit den Verbund der textilen Auflage auf dem Träger während der Lagerung und der Tragedauer. D.h. die Klebeigenschaften zwischen textilen Auflage und Klebeschicht des Trägers werden beibehalten. Zweiter Zweck ist die Wasserdampfundurchlässigkeit. Durch die dichte Folie kann erst das erwünschte feuchte Wundmilieu im Wundbereich erzeugt werden.
Von Vorteil dabei ist, dass diese feuchte Wundheilung nur im Bereich der Wunde (textile Wundauflage) erzeugt wird und nicht im Bereich der klebenden Randschicht des Trägers. Somit weist die wasserdampfundurchlässige Barriereschicht die gleiche Größe wie die textile Auflage auf und die Trägerschicht ragt bevorzugt über die Barriereschicht hinaus. Die vorteilhaft klebende Randschicht ist möglichst wasserdampfdurchlässig, um gute Klebeigenschaften des gesamten Pflasters zu gewährleisten.
Eine Mazeration wird durch diesen erfindungsgemäß vorteilhaften Aufbau vermieden. Der Wundauflagenrand, die Dicke der Wundauflage, weist keine wasserdampfundurchlässige Barriere auf. Dieser Wundauflagenrand ermöglicht so eine gewisse Austrocknung der Wundauflage, genug um Mazeration vorzubeugen, aber nicht genug um eine feuchte Wundheilung zu schaffen.

Bevorzugt ist die Menge an Salbe so gewählt, dass die textile Auflage noch saugfähig ist und Wundsekret oder sonstige Körperflüssigkeiten aufnehmen kann, was bei den bekannten Pflasterkombinationen mit Creme bzw. Salbe nicht gegeben ist. Vorteilhaft ist somit das Verhältnis von Menge (Masse in mg) an Salbe zu Größe (Fläche im mm²) der textilen Auflage im Bereich von 0,3 bis 0,01, bevorzugt 0,2 bis 0,01, insbesondere bevorzugt im Bereich von 0,05 bis 0,16 (in mg/mm²) zu wählen. Beispielsweise sind vorteilhaft 75 mg Salbe in eine textile Wundauflage der Fläche 18 x 30 mm² inkorporiert, was zu einem Verhältnis von etwa 0,138 führt.

Die inkorporierte Salbe füllt die Leerräume der textilbasierten Wundauflage dabei vorteilhaft nicht vollständig aus.
Damit ist neben des feuchten Wundmilieus, der Salbenapplikation aus der Auflage gleichzeitig auch die Aufnahme von Exsudat gewährleistet.

Die wasserdampfdurchlässige Trägerschicht mit Klebmasse ermöglicht, dass das erfindungsgemäße Pflaster nicht durch Feuchtigkeitsstau im Haftvermögen beeinträchtigt wird bzw. die Haut durch Mazeration geschädigt wird.
Die erfindungsgemäße Kombination aus einer wasserdampfundurchlässigen Barriereschicht über der Wunde aber mit einem offenen Randbereich ermöglicht eine feuchte Wundheilung unter dem Pflaster. Die Salbe legt sich auf die Wunde und bietet dadurch extra Schutz vor Austrocknung.

Diese Insellösung stellt zudem eine preiswerte Möglichkeit dar,
Standardpflasterherstellverfahren für die Herstellung erfindungsgemäßer Auflagen zu nutzen.
Die erfindungsgemäßen Auflagen können an Standard Einzelpflastermaschinen hergestellt werden. Träger, Barriereschicht, textile Auflage und Salbe werden mittels einer Maschine zusammengebracht bzw. ausgestanzt/geschnitten und bevorzugt als Einzelpflaster eingesiegelt. Die Maschine ist dabei vorteilhaft so umgebaut, dass kein Druck auf der textilen Auflage entsteht um ein Ausdrücken der Salbe aus der Auflage zu vermeiden.
Die Salbe (Creme/Ointment) wird in-line während der Einzelpflasterherstellung auf die textile Auflage aufgebracht.

Das Verfahren zur Herstellung der erfindungsgemäßen Auflagen umfassend bevorzugt ein Trägermaterial, eine wasserdampfundurchlässige Barriereschicht und eine daran anliegende Wundauflage auf Textilbasis erfolgt indem die Textilauflage zunächst mit einer Salbe beaufschlagt wird. Die Salbe wird vor der Beaufschlagung weitestgehend entgast und in ihrer Viskosität erniedrigt, so dass sie nach der Beaufschlagung in die textile Auflage inkorporieren kann.
Damit ist erfindungsgemäß erstmals eine Kombination aus Wundauflage in Kombination mit Wundpflege über einen Salbengrundlage gegeben. Die textile Wundauflage kann so auch weiterhin Wundexsudat oder Blut aufnehmen.

Das erfindungsgemäße Verfahren ermöglicht eine schnelle, ökonomische Herstellung von Pflastern, deren Wundauflage vollständig oder partiell mit einer Creme/Salbe oder einer ähnlichen Zubereitung dotiert ist. Eine besondere Herausforderung bestand darin, die Dotierung möglichst genau hinsichtlich der aufzubringenden Menge und der räumlichen Ausdehnung und unter möglichst geringer thermischer Belastung des Mediums durchzuführen. Die Salbe/Creme wird daher vor dem Aufbringen auf die textile Auflage dahingehend präpariert, dass die Salbe/Creme ohne Gaseinschlüsse bei der Dotierung zur Verfügung steht. Die Verarbeitung der Salbe/Creme erfolgt dabei vorteilhaft in drei Schritten.

Im ersten Schritt wird die Salbe aus einem Transportbehälter (z.B. Fass) mit Hilfe eines Stempels bei Raumtemperatur ausgetragen. Im zweiten Schritt wird eine geringe Menge der Salbe in einem Zwischenbehälter erwärmt und verflüssigt. Das Verflüssigen und die Geometrie des Zwischenbehälters ermöglicht eine gezielte Entlüftung der Salbe und damit außerdem die hohe Dosiergenauigkeit des flüssigen Mediums. Die Salbe wird vorteilhaft erwärmt um die Viskosität zu erniedrigen und ein Einfließen in die textile Auflage zu gewährleisten.
Durch die Koppelung von Schritt eins und zwei wird die Menge der aufgeschmolzenen Salbe minimal gehalten. Dadurch ist die zeitliche Temperaturbelastung gering. Im dritten Schritt wird die Salbe über Zahnradpumpen dem Auftragskopf zugeführt. Über den Auftragskopf wird eine genau definierte Menge Salbe über Kontakt, beispielsweise dem Abstreifen, oder durch zielgenaues Spritzen/Sprühen auf die textile Wundauflage aufgetragen. Durch dieses Verfahren ist auch ein gezieltes Aufbringen auf räumlich begrenzte Areale der Wundauflage möglich.

Durch diese Verarbeitungsweise wird gewährleisten, dass die Salbe ohne Gaseinschlüsse blasenfrei und damit mit sehr hoher Genauigkeit aufgebracht wird und außerdem die Temperaturbelastung der Salbe stark reduziert ist. Die Menge Salbe pro Pflaster und die räumliche Orientierung der Salbe auf der Wundauflage ist somit reproduzierbar und genau einstellbar. Dieses Vorgehen ist insbesondere wichtig damit keine Schädigung der Salbe oder temperaturempfindlicher Inhaltsstoffe erfolgen kann.

Abschließend kann das Pflaster mit einem klebstoffabweisenden Abdeckmaterial, wie silikonisierter Folie, eingedeckt werden.

Erfindungsgemäß ist die Salbe damit in der Wundauflage inkorporiert und nicht, wie im Stand der Technik beschrieben, auf einer Schicht, d.h. oberflächig, aufgetragen.
Inkorporieren bedeutet erfindungsgemäß beinhalten, einverleiben. D.h. die Salbe ist in der Wundauflage und nicht oberflächlich aufgetragen, wie es in der Abbildung 1 auch dargestellt ist.

Die Salbe ist bevorzugt so dickflüssig, dass sie bei Raumtemperatur, in der Wundauflage verbleibt. Zur Viskositätserhöhung können der Salbe Fette, Wachse oder andere Stoffe zugesetzt sein.
Die Wundauflage ist vorteilhaft mit Salbe nicht gesättigt, was auch eine noch vorhandene Saugfähigkeit ermöglicht.
Die erfindungsgemäße Haut- oder Wundauflage ist vorteilhaft nach obigem Verfahren herstellbar und unterscheidet sich dadurch gegenüber den Auflagen des Standes der Technik, dadurch, dass
- die inkorporierte Salbe nicht nur oberflächig auf der Wundauflage liegt
- die Wundauflage Blut und/oder Wundexudat aufnehmen kann.

Die Salbe ist vorteilhaft als fettig zu charakterisieren damit sie zur feuchten Wundheilung beiträgt und enthält bevorzugt kein Wasser, da ansonsten das Wasser bei der Auftragung und Lagerung austrocknen würde. Eine wasserhaltige Salbe könnte auch zu mikrobiologischer Instabilität führen und müsste konserviert sein, was bei einer Wundapplikation nicht gewünscht ist. Die Salbe bildet so dann zusammen mit der Barriereschicht eine deckende Schicht auf der Wunde und gewährleistet das wundheilungsfördernde feuchte Milieu.

Beim Applizieren der Auflage wird durch den Applikationsdruck oder beim Befestigen auf der Haut die Salbe aus der textilen Auflage auf die Haut abgegeben und kann hier pflegend oder wundheilend wirken.

### Beispiel 1:

Pflaster bestehend aus einem Vliesträger, Hersteller Sontara Vlies von Dupont, mit einer Akrylatklebemasse von BASF (CAS No 25119-83-9) beschichtet. Die Barriereschicht besteht aus 12 µm PET-Folie Hostaphan und die Wundauflage aus einem 126 g/m² drylaid PP Vlies, durch einen aliphatischen PUR (Polyurethan)-Kleber verbunden. Als Salbe wird Vaseline von der Firma Merkur verwendet. Die textile Wundauflage ist 12x25 mm² groß und eine Crememenge von 0,03 g wird unter Heizung auf die textile Wundauflage durch drei Düsenöffnungen appliziert. Die Vaseline zieht so in die textile Wundauflage ein und das Pflaster wird nach der Herstellung einzeln eingesiegelt.

### Beispiel 2:

Pflaster bestehend aus einem Folienträger, perforiertem PE, mit einer Akrylatklebemasse von BASF (CAS No 25119-83-9) beschichtet. Die Barriereschicht besteht aus 12 µm PET-Folie Hostaphan und die textile Wundauflage aus einem 126 g/m² drylaid PP Vlies, durch einen aliphatischen PUR-Kleber verbunden. Als Salbe wird Aquaphor Original Ointment von der Firma Beiersdorf AG verwendet. Die textile Wundauflage ist 18x30 mm² groß und eine Crememenge von 0,075 g wird unter Heizung auf die textile Wundauflage durch Streichverfahren appliziert. Das Pflaster wird nach der Herstellung einzeln eingesiegelt.

## Patentansprüche

1. Wund- oder Hautauflage umfassend
- eine wasserdampfundurchlässige Barriereschicht und
- eine daran anliegende Auflage auf Textilbasis, in die eine Salbe oder Creme inkorporiert ist.

2. Wund- oder Hautauflage nach Anspruch 1 umfassend zusätzlich ein Trägermaterial.

3. Auflage nach Anspruch 2 **dadurch gekennzeichnet, dass** das Trägermaterial wasserdampfdurchlässig ist.

4. Auflage nach Anspruch 2 oder 3 **dadurch gekennzeichnet, dass** das Trägermaterial mit einer Klebmasse beaufschlagt ist.

5. Auflage nach einem der vorstehenden Ansprüche **dadurch gekennzeichnet, dass** die Salbe oder Creme wasserfrei ist.

6. Auflage nach einem der vorstehenden Ansprüche **dadurch gekennzeichnet, dass** die Salbe Petrolatum oder Mischungen umfassend Petrolatum umfasst.

7. Auflage nach einem der vorstehenden Ansprüche **dadurch gekennzeichnet, dass** die Textilauflage oder Salbe Wirkstoffe, natürliche Zusatzstoffe und/oder viskositätserhöhende Stoffe umfasst.

8. Auflage nach Anspruch 7 **dadurch gekennzeichnet, dass** ein oder mehrere Wirkstoffe gewählt werden aus der Gruppe der-Analgestika, Antirheumatika, Desinfizientia, Antiseptika, Antibiotika, Antimykotika und/oder Lokalanesthetika.

9. Auflage nach Anspruch 8 **dadurch gekennzeichnet, dass** ein oder mehrere Wirkstoffe gewählt werden aus der Gruppe der Lokalanesthetika.

10. Auflage nach Anspruch 7, umfassend mindestens einen Wirkstoff gewählt aus der Gruppe der entzündungshemmenden oder wundheilungsfördernden Wirkstoffe.

11. Auflage nach einem der vorstehenden Ansprüche **dadurch gekennzeichnet, dass** die Wirkstoffe zu einem Anteil von 0,001 - 10 Gew.%, bevorzugt 0,01 - 5 Gew.%, insbesondere bevorzugt 0,05-1 Gew.%, bevorzugt 0,01-0,5 Gew.%, bezogen auf die Gesamtmasse der Textilauflage oder Salbe, umfasst sind.

12. Auflage nach einem der vorstehenden Ansprüche **dadurch gekennzeichnet, dass** die Textilauflage ein Vlies aus Polypropylen, Viskose oder Polyester ist.

13. Auflage nach einem der vorstehenden Ansprüche **dadurch gekennzeichnet, dass** die Barriereschicht aus Polyethylentherephthalat oder Polyamid besteht.

14. Auflage nach einem der vorstehenden Ansprüche **dadurch gekennzeichnet, dass** das Verhältnis von Masse der Salbe (in mg) zu Fläche der Textilauflage (in mm²) im Bereich von 0,3 bis 0,01, insbesondere 0,2 bis 0,01, bevorzugt im Bereich von 0,05 bis 0,16 gewählt wird.

15. Auflage nach einem der vorstehenden Ansprüche **dadurch gekennzeichnet, dass** die wasserdampfundurchlässige Barriereschicht die gleiche Größe wie die Textilauflage aufweist und gegebenenfalls die Trägerschicht über die Barriereschicht hinausragt.

16. Verfahren zur Herstellung einer Wund- oder Hautauflage umfassend ein Trägermaterial, eine wasserdampfundurchlässige Barriereschicht und eine daran anliegende Auflage auf Textilbasis, die mit einer Salbe beaufschlagt wird **dadurch gekennzeichnet, dass** die Salbe vor der Beaufschlagung weitestgehend entgast und in ihrer Viskosität erniedrigt wird und nach der Beaufschlagung in die Textilauflage inkorporiert.

17. Verfahren nach Anspruch 16 **dadurch gekennzeichnet, dass** die Salbe vor dem Beaufschlagen in einem Zwischenbehälter erwärmt wird.

18. Verfahren nach Anspruch 16 oder 17 **dadurch gekennzeichnet, dass** die Salbe in einer Menge (in mg) aufgebracht wird, so dass sich ein Verhältnis von Masse der Salbe (in mg) zur Fläche der Textilauflage (in mm²) im Bereich von 0,3 bis 0,01 ergibt.

19. Verfahren nach Anspruch 16, 17 oder 18 **dadurch gekennzeichnet, dass** die wasserdampfundurchlässige Barriereschicht die gleiche Größe wie die Textilauflage aufweist und die Trägerschicht über die Barriereschicht hinausragt.

20. Wund- oder Hautauflage erhältlich nach einem der Ansprüche 16 bis 19.

21. Verwendung einer Wund- oder Hautauflage umfassend eine wasserdampfundurchlässige Barriereschicht und eine daran anliegende Auflage auf Textilbasis, in die eine Salbe oder Creme inkorporiert ist, wobei die Salbe oder die Auflage einen oder mehrere Wirkstoffe umfasst gewählt aus der Gruppe der Analgestika, Antirheumatika, Desinfizientia, Antiseptika, Antibiotika, Antimykotika und/oder Lokalanesthetika zur Herstellung eines Analgestikum, Antirheumatikum, Desinfizientium, Antiseptikum, Antibiotikum, Antimykotikum und/oder Lokalanesthetikum.

22. Verwendung nach Anspruch 21 **dadurch gekennzeichnet, dass** ein oder mehrere Wirkstoffe aus der Gruppe der Lokalanesthetika gewählt werden zur Herstellung eines Lokalanesthetikum.

## Claims

1. Wound or skin dressing comprising
- a water-vapour-impermeable barrier layer and
- a textile-based lining which is in contact therewith and in which an ointment or cream is incorporated.

2. Wound or skin dressing according to Claim 1, comprising additionally a support material.

3. Dressing according to Claim 2, **characterized in that** the support material is water-vapour-permeable.

4. Dressing according to Claim 2 or 3, **characterized in that** an adhesive is applied to the support material.

5. Dressing according to any of the preceding claims, **characterized in that** the ointment or cream is anhydrous.

6. Dressing according to any of the preceding claims, **characterized in that** the ointment comprises petrolatum or mixtures comprising petrolatum.

7. Dressing according to any of the preceding claims, **characterized in that** the textile lining or ointment comprises active ingredients, natural additives and/or viscosity-increasing substances.

8. Dressing according to Claim 7, **characterized in that** one or more active ingredients are selected from the group of analgesics, anti-rheumatics, disinfectants, antiseptics, antibiotics, antimycotics and/or local anaesthetics.

9. Dressing according to Claim 8, **characterized in that** one or more active ingredients are selected from the group of local anaesthetics.

10. Dressing according to Claim 7, comprising at least one active ingredient selected from the group of anti-inflammatory or wound-healing-promoting active ingredients.

11. Dressing according to any of the preceding claims, **characterized in that** the active ingredients are included to a proportion of 0.001-10% by weight, preferably 0.01-5% by weight, especially preferably 0.05-1% by weight, preferably 0.01-0.5% by weight, based on the total mass of the textile lining or ointment.

12. Dressing according to any of the preceding claims, **characterized in that** the textile lining is a non-woven composed of polypropylene, viscose or polyester.

13. Dressing according to any of the preceding claims, **characterized in that** the barrier layer consists of polyethylene terephthalate or polyamide.

14. Dressing according to any of the preceding claims, **characterized in that** the ratio of ointment mass (in mg) to textile lining area (in mm²) is selected within the range of from 0.3 to 0.01, more particularly from 0.2 to 0.01, preferably within the range of from 0.05 to 0.16.

15. Dressing according to any of the preceding claims, **characterized in that** the water-vapour-impermeable barrier layer has the same size as the textile lining and optionally the support layer projects beyond the barrier layer.

16. Method for producing a wound or skin dressing comprising a support material, a water-vapour-impermeable barrier layer and a textile-based lining which is in contact therewith and to which an ointment is applied, **characterized in that** the ointment is outgassed to the greatest possible extent and lowered in its viscosity before the application and incorporated in the textile lining after the application.

17. Method according to Claim 16, **characterized in that** the ointment is heated in an intermediate vessel before the application.

18. Method according to Claim 16 or 17, **characterized in that** the ointment is applied in an amount (in mg) yielding a ratio of ointment mass (in mg) to textile lining area (in mm²) within the range of from 0.3 to 0.01.

19. Method according to Claim 16, 17 or 18, **characterized in that** the water-vapour-impermeable barrier layer has the same size as the textile lining and the support layer projects beyond the barrier layer.

20. Wound or skin dressing obtainable according to any of Claims 16 to 19.

21. Use of a wound or skin dressing comprising a water-vapour-impermeable barrier layer and a textile-based lining which is in contact therewith and in which an ointment or cream is incorporated, wherein the ointment or the lining comprises one or more active ingredients selected from the group of analgesics, anti-rheumatics, disinfectants, antiseptics, antibiotics, antimycotics and/or local anaesthetics for the production of an analgesic, anti-rheumatic, disinfectant, antiseptic, antibiotic, antimycotic and/or local anaesthetic.

22. Use according to Claim 21, **characterized in that** one or more active ingredients are selected from the group of local anaesthetics for the production of a local anaesthetic.

## Revendications

1. Pansement pour les plaies ou pour la peau, comprenant :
- une couche de barrière imperméable à la vapeur d'eau et
- un revêtement à base de textile disposé sur celle-ci, dans lequel un onguent ou une crème est incorporé.

2. Pansement pour les plaies ou pour la peau selon la revendication 1, comprenant en outre un matériau support.

3. Pansement selon la revendication 2, **caractérisé en ce que** le matériau support est perméable à la vapeur d'eau.

4. Pansement selon la revendication 2 ou 3, **caractérisé en ce que** le matériau support est chargé avec une masse adhésive.

5. Pansement selon l'une quelconque des revendications précédentes, **caractérisé en ce que** l'onguent ou la crème est anhydre.

6. Pansement selon l'une quelconque des revendications précédentes, **caractérisé en ce que** l'onguent comprend de la vaseline ou des mélanges comprenant de la vaseline.

7. Pansement selon l'une quelconque des revendications précédentes, **caractérisé en ce que** le revêtement textile ou l'onguent comprend des agents actifs, des additifs naturels et/ou des substances augmentant la viscosité.

8. Pansement selon la revendication 7, **caractérisé en ce qu'**un ou plusieurs agents actifs sont choisis dans le groupe constitué par les analgésiques, les agents antirhumatismaux, les désinfectants, les antiseptiques, les antibiotiques, les antimycotiques et/ou les anesthésiques locaux.

9. Pansement selon la revendication 8, **caractérisé en ce qu'**un ou plusieurs agents actifs sont choisis dans le groupe des anesthésiques locaux.

10. Pansement selon la revendication 7, comprenant au moins un agent actif choisi dans le groupe des agents actifs anti-inflammatoires et cicatrisants.

11. Pansement selon l'une quelconque des revendications précédentes, **caractérisé en ce que** les agents actifs sont compris jusqu'en une proportion de 0,001 à 10 % en poids, de préférence de 0,01 à 5 % en poids, de manière particulièrement préférée de 0,05 à 1 % en poids, de préférence de 0,01 à 0,5 % en poids, par rapport à la masse totale du revêtement textile ou de l'onguent.

12. Pansement selon l'une quelconque des revendications précédentes, **caractérisé en ce que** le revêtement textile est un non-tissé en polypropylène, viscose ou polyester.

13. Pansement selon l'une quelconque des revendications précédentes, **caractérisé en ce que** la couche de barrière est constituée de polyéthylène téréphtalate ou de polyamide.

14. Pansement selon l'une quelconque des revendications précédentes, **caractérisé en ce que** le rapport entre la masse de l'onguent (en mg) et la surface du revêtement textile (en mm²) est choisi dans la plage allant de 0,3 à 0,01, notamment de 0,2 à 0,01, de préférence dans la plage allant de 0,05 à 0,16.

15. Pansement selon l'une quelconque des revendications précédentes, **caractérisé en ce que** la couche de barrière imperméable à la vapeur d'eau présente la même taille que le revêtement textile et la couche support dépasse éventuellement au-dessus de la couche de barrière.

16. Procédé de fabrication d'un pansement pour les plaies ou pour la peau comprenant un matériau support, une couche de barrière imperméable à la vapeur d'eau et un revêtement à base de textile disposé sur celle-ci, qui est chargé avec un onguent, **caractérisé en ce que** l'onguent est essentiellement dégazé avant le chargement et sa viscosité est réduite, et incorporé après le chargement dans le revêtement textile.

17. Procédé selon la revendication 16, **caractérisé en ce que** l'onguent est chauffé dans un contenant intermédiaire avant le chargement.

18. Procédé selon la revendication 16 ou 17, **caractérisé en ce que** l'onguent est appliqué en une quantité (en mg) telle qu'un rapport entre la masse de l'onguent (en mg) et la surface du revêtement textile (en mm²) soit dans la plage allant de 0,3 à 0,01.

19. Procédé selon la revendication 16, 17 ou 18, **caractérisé en ce que** la couche de barrière imperméable à la vapeur d'eau présente la même taille que le revêtement textile et la couche support dépasse au-dessus de la couche de barrière.

20. Pansement pour les plaies ou pour la peau pouvant être obtenu selon l'une quelconque des revendications 16 à 19.

21. Utilisation d'un pansement pour les plaies ou pour la peau comprenant une couche de barrière imperméable à la vapeur d'eau et un revêtement à base de textile disposé sur celle-ci, dans lequel un onguent ou une crème est incorporé, l'onguent ou le revêtement comprenant un ou plusieurs agents actifs choisis dans le groupe constitué par les analgésiques, les agents antirhumatismaux, les désinfectants, les antiseptiques, les antibiotiques, les antimycotiques et/ou les anesthésiques locaux pour la fabrication d'un analgésique, d'un agent antirhumatismal, d'un désinfectant, d'un antiseptique, d'un antibiotique, d'un antimycotique et/ou d'un anesthésique local.

22. Utilisation selon la revendication 21, **caractérisée en ce qu'**un ou plusieurs agents actifs choisis dans le groupe des anesthésiques locaux sont choisis pour la fabrication d'un anesthésique local.
